# EUROPEAN PATENT APPLICATION

(11) **EP 3 760 205 A1**
(43) Date of publication of application: **06.01.2021**
(21) Application number: 19760215.4
(22) Date of filing: 27.02.2019
(51) Int. Cl.: A61K 31/496, A61K 9/10, A61K 47/02, A61K 47/18, A61K 47/26, A61K 47/32, A61K 47/38, A61P 25/00, A61P 25/18, A61P 25/24, A61P 25/28

(54) **AQUEOUS SUSPENSION-TYPE PHARMACEUTICAL PREPARATION**

(30) Priority: 28.02.2018 JP 2018035595
(71) Applicant: Sumitomo Dainippon Pharma Co., Ltd., Chuo-ku Osaka-shi Osaka 541-8524 (JP)
(72) Inventor: TSUZUKU, Takuma, Tokyo 104-8356 (JP)
(74) Representative: Vossius & Partner Patentanwälte Rechtsanwälte mbB
(86) International application number: PCT/JP2019/007422
(87) International publication number: WO 2019/167977

(57) **Abstract**

The present invention provides an aqueous suspension pharmaceutical preparation with a pH of 2.5 - 5.5, comprising the following (1) to (4):
(1) (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1), a pharmaceutically acceptable acid addition salt thereof, or a mixture thereof,
(2) one or more chlorides selected from an inorganic chloride or a quaternary ammonium chloride having 4 to 12 carbon atoms,
(3) a dispersant, and
(4) water,
which exhibits the solubility in the stomach depending on the property of a drug without producing rapid dissolution and absorption even when the preparation is administered orally and can inhibit the particle growth and the aggregation of particles.

## Description

### TECHNICAL FIELD

The present invention relates to an aqueous suspension pharmaceutical preparation. In detail, the present invention relates to an aqueous suspension pharmaceutical preparation comprising (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindol-1,3-dione (hereinafter also referred to as "Compound 1"), a pharmaceutically acceptable acid addition salt thereof, or a mixture thereof (hereinafter also referred to as "Compound 1 or salt thereof") which is suspended in a solvent including water. Preferably, the present invention relates to the preparation for using as medicine.

### BACKGROUND ART

It is well known that (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindol-1,3-dione (Compound 1) is a compound used as an atypical antipsychotic agent, and the compound is useful in the treatment of a psychiatric disease such as schizophrenia, bipolar disorder, and depression (e.g. Patent Documents 1 and 2).

In general, an oral solid pharmaceutical preparation such as tablet and capsule has been widely used in the treatment of various diseases because it is easy for patients to take medicine at home. On the other hand, there are some situations which cannot be sufficiently treated by only the use of an oral solid preparation, in case of, for example, the administration to a patient suffering from acute schizophrenia, the administration to a patient in particular need of adjusting a dosage like children and senior people, the administration to a patient who refuses to take a drug, and the administration to a patient who has difficulty in swallowing, because the psychiatric disease such as schizophrenia causes various symptoms for each patient. Recently, the number of senior people is increasing, and thus it has been strongly desired in the medical field to provide an oral solution and an injection as a preparation with the improved medication compliance that reduces the burden for the senior patient's group.

As an oral solution of Compound 1, a solution preparation comprising N-[4-[4-(1,2-benzisothiazol-3-yl)-1-piperazinyl]-(2R,3R)-2,3-tetramethylene-butyl]-(1'R,2'S,3'R,4'S)-2,3-bicyclo[2,2,1]heptanedicarboxyimide hydrochloride which is Compound 1 hydrochloride as an active ingredient and at least one substance selected from benzyl alcohol, N,N-dimethylacetamide, lactic acid or propylene glycol has ever been reported (Patent Document 3). However, Patent Document 3 focuses on solubilizing the compound at high concentration. Patent Document 3 discloses that the solubility and stability of the solution preparation are characteristic, but does not disclose that the properties are studied from pharmacokinetic points. In addition, Patent Document 3 neither discloses nor suggests any suspension preparation.

Patent Document 4 discloses a composition comprising N-[4-[4-(1,2-benzisothiazol-3-yl)-1-piperazinyl]-(2R,3R)-2,3-tetramethylene-butyl]-(1'R,2'S,3'R,4'S)-2,3-bicyclo[2,2,1]heptanedicarboxyimide which is Compound 1, or a pharmaceutically acceptable acid addition salt thereof in the form of suspended particle, and more specifically a sustained-release preparation for injection which maintains an effective blood level of the compound. However, Patent Document 4 does not disclose that the preparation is administered orally. That is, Patent Document 4 neither discloses nor suggests any property such as solubility in the stomach and absorbability when the preparation is administered orally.

### PRIOR ART DOCUMENTS

### PATENT DOCUMENTS

Patent Document 1: JP 2800953 B (US 5532372 A)
Patent Document 2: WO 2005/009999
Patent Document 3: WO 2006/134864
Patent Document 4: WO 2012/053654

### SUMMARY OF INVENTION

### (PROBLEM TO BE SOLVED BY THE INVENTION)

When an aqueous preparation comprising a drug with low solubility is prepared, a method of solubilizing the drug with an additive such as solubilizer and surfactant or a method of suspending the drug under the condition with low solubility is basically selected.

An aqueous preparation in which a drug is solubilized has some merits, e.g., the preparation can be relatively easy to be taken for a patient who has difficulty in swallowing and can be taken with other drink. However, in the preparation, the drug in dissolved state easily interacts with bitter taste receptors on the tongue, and thus when a compound with strong bitterness such as Compound 1 is used, there were some problems, e.g., the mental burden for a patient when taking the preparation tends to increase because it is difficult to mask bad taste of such compound, and/or it is difficult to maintain the quality of a drug in dissolved state as medicine because such drug easily reduces the chemical stability. In addition, it has been thought that when a drug in dissolved state is administered orally, the drug is easily absorbed in short time to rapidly increase the blood level of the drug, and thus the increased blood level may lead to any disadvantage such as the occurrence of side effects.

On the other hand, an aqueous suspension preparation in which a drug is suspended has some merits, e.g., the preparation can inhibit bad taste derived from the drug and easily maintain the chemical stability of the drug under the condition with low solubility. However, in the preparation, the drug solubility in the stomach varies depending on factors such as the particle size and the surface state of a drug in the form of suspended particle or the effect of a surfactant when the preparation comprises the surfactant. Hence, it has been thought that such preparation have some problems, e.g., it is difficult to predict the absorbability of a drug. Actually, when a poorly water-soluble basic drug such as Compound 1 is suspended in water, it has been thought that it is basically effective to adjust the pH of the suspension to the neutral or weakly basic to inhibit bad taste of the drug and maintain the chemical stability of the drug. However, it has been suggested that a suspension preparation comprising Compound 1 prepared under such condition unexpectedly shows higher dissolution in the stomach as compared to tablet and may occur undesired side effects. In view of the suggestion, the major problem in the development of an aqueous preparation was to minimize the risk of the effectiveness and stability by the control of the dissolution in the stomach.

A basic drug such as Compound 1 is dissolved in the low pH environment in the stomach, and then the drug is transferred to the small intestine with high pH and absorbed. In such case, a phenomenon such as supersaturated dissolution produced in either or both of the pH environments keeps the concentration of a drug high, and thus produces unexpected absorption rate of the drug. As a result, the initial Cmax value is increased and the increased Cmax value may lead to any disadvantage such as the occurrence of side effects.

The present invention has been studied considering the above situation, and an object of the present invention is to provide an aqueous suspension pharmaceutical preparation comprising Compound 1 or salt thereof as an active ingredient wherein the aqueous suspension pharmaceutical preparation exhibits the solubility in the stomach depending on the property of a drug without producing rapid dissolution and absorption even when the preparation is administered orally.

### (MEANS FOR SOLVING THE PROBLEMS)

The present inventors have extensively studied to reach the above object, and then have found that when the pH range which is not basically selected by those skilled in the art, that is, the weakly acidic pH range in which the solubility of Compound 1 in water is relatively high is selected and the preparation comprises chloride ion together, the dissolution amount of Compound 1 or salt thereof in suspension (the dissolution fractional concentration in preparation) is not unexpectedly increased so much, and that when a drug is added to the acidic solution that imitates the stomach (e.g. 1st fluid for dissolution test described in the Japanese Pharmacopoeia 17th Edition), the dissolution rate of the drug is inhibited, and thus rapid absorption of the drug can be inhibited and the particle growth and the aggregation of particles can be inhibited. Based upon the new findings, the present invention has been completed.

The present invention provides inventions described below.
[Item 1] An aqueous suspension pharmaceutical preparation with a pH of 2.5 - 5.5, comprising the following (1) to (4):
   (1) (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1), a pharmaceutically acceptable acid addition salt thereof, or a mixture thereof,
   (2) one or more chlorides selected from an inorganic chloride or a quaternary ammonium chloride having 4 to 12 carbon atoms,
   (3) a dispersant, and
   (4) water.
[Item 2] The pharmaceutical preparation according to the item 1, wherein the pH is 3.0 - 5.5.
[Item 3] The pharmaceutical preparation according to the item 1, wherein the pH is 3.0 - 5.0.
[Item 4] The pharmaceutical preparation according to any one of the items 1 to 3, which comprises at least one ingredient selected from the group consisting of a cellulose derivative, a sucrose fatty acid ester, and polyvinyl alcohol as the dispersant.
[Item 5] The pharmaceutical preparation according to the item 4, which comprises a cellulose derivative as the dispersant.
[Item 6] The pharmaceutical preparation according to any one of the items 1 to 3, which comprises at least one ingredient selected from the group consisting of hypromellose, hydroxypropylcellulose, and hydroxyethyl cellulose as the dispersant.
[Item 7] The pharmaceutical preparation according to the item 6, which comprises hypromellose as the dispersant.
[Item 8] The pharmaceutical preparation according to any one of the items 1 to 7, which comprises at least one ingredient selected from the group consisting of sodium chloride, potassium chloride, iron (II) chloride, calcium chloride, magnesium chloride, and choline chloride as the chloride.
[Item 9] The pharmaceutical preparation according to the item 8, which comprises at least one ingredient selected from the group consisting of sodium chloride, potassium chloride, and choline chloride as the chloride.
[Item 10] The pharmaceutical preparation according to the item 8, which comprises at least one ingredient selected from the group consisting of sodium chloride and choline chloride as the chloride.
[Item 11] The pharmaceutical preparation according to the item 8, which comprises sodium chloride as the chloride.
[Item 12] The pharmaceutical preparation according to any one of the items 1 to 11, wherein the concentration of chloride ion is about 0.015 - about 1 mol/L, provided that the chloride ion does not comprise the chloride ion derived from a salt of Compound 1.
[Item 13] The pharmaceutical preparation according to the item 12, wherein the concentration of chloride ion is about 0.05 - about 1 mol/L.
[Item 14] The pharmaceutical preparation according to the item 12, wherein the concentration of chloride ion is about 0.10 - about 1 mol/L.
[Item 15] The pharmaceutical preparation according to any one of the items 1 to 14, wherein the content of Compound 1, a pharmaceutically acceptable acid addition salt thereof, or a mixture thereof is about 5 - about 120 mg/mL in terms of Compound 1 hydrochloride.
[Item 16] The pharmaceutical preparation according to any one of the items 1 to 14, wherein the content of Compound 1, a pharmaceutically acceptable acid addition salt thereof, or a mixture thereof is about 10 - about 100 mg/mL in terms of Compound 1 hydrochloride.
[Item 17] The pharmaceutical preparation according to any one of the items 1 to 14, wherein the content of Compound 1, a pharmaceutically acceptable acid addition salt thereof, or a mixture thereof is about 20 - about 80 mg/mL in terms of Compound 1 hydrochloride.
[Item 18] The pharmaceutical preparation according to any one of the items 1 to 17, wherein Compound 1, a pharmaceutically acceptable acid addition salt thereof, or a mixture thereof is Compound 1 hydrochloride.
[Item 19] The pharmaceutical preparation according to any one of the items 1 to 18 for oral administration.
[Item 20] The pharmaceutical preparation according to any one of the items 1 to 19, wherein the maximum dissolution amount of Compound 1, a pharmaceutically acceptable acid addition salt thereof, or a mixture thereof at 0 - 30 minutes after the start of the test performed under the following test condition according to the dissolution test (the paddle method) described in the Japanese Pharmacopoeia 17th Edition is about 120 µg/mL or less in terms of Compound 1 hydrochloride.

### [Test Condition]

Test solution: 1st fluid for dissolution test described in the Japanese Pharmacopoeia 17th Edition Volume of 1st fluid for dissolution test: 250 mL Temperature of test solution: 36.5 - 37.5°C
Rotation speed: 50 rpm
Test preparation: Preparation comprising 40 mg of Compound 1, a pharmaceutically acceptable acid addition salt thereof, or a mixture thereof in terms of Compound 1 hydrochloride Sampling time of test solution: 10 minutes, 20 minutes and 30 minutes after the start of test
[Item 21] The pharmaceutical preparation according to the item 20, wherein the maximum dissolution amount of Compound 1, a pharmaceutically acceptable acid addition salt thereof, or a mixture thereof at 0 - 30 minutes after the start of the test is about 110 µg/mL or less in terms of Compound 1 hydrochloride.
[Item 22] The pharmaceutical preparation according to the item 20, wherein the maximum dissolution amount of Compound 1, a pharmaceutically acceptable acid addition salt thereof, or a mixture thereof at 0 - 30 minutes after the start of the test is about 100 µg/mL or less in terms of Compound 1 hydrochloride.
[Item 23] The pharmaceutical preparation according to any one of the items 1 to 22, wherein the D90 of the suspended particle is about 1 to about 30 µm.
[Item 24] The pharmaceutical preparation according to any one of the items 1 to 22, wherein the D90 of the suspended particle is about 1 to about 20 µm.
[Item 25] The pharmaceutical preparation according to any one of the items 1 to 22, wherein the D90 of the suspended particle is about 1 to about 15 µm.
[Item 26] The pharmaceutical preparation according to any one of the items 1 to 22, wherein the D50 of the suspended particle is about 0.1 to about 13 µm.
[Item 27] The pharmaceutical preparation according to any one of the items 1 to 22, wherein the D50 of the suspended particle is about 0.1 to about 10 µm.
[Item 28] The pharmaceutical preparation according to any one of the items 1 to 22, wherein the D50 of the suspended particle is about 0.1 to about 7 µm.
[Item 29] The pharmaceutical preparation according to any one of the items 1 to 28, wherein the concentration of Compound 1, a pharmaceutically acceptable acid addition salt thereof, or a mixture thereof dissolved in the preparation (the dissolution fractional concentration in the preparation) is about 360 µg/mL or less in terms of Compound 1 hydrochloride.
[Item 30] The pharmaceutical preparation according to any one of the items 1 to 28, wherein the concentration of Compound 1, a pharmaceutically acceptable acid addition salt thereof, or a mixture thereof dissolved in the preparation (the dissolution fractional concentration in the preparation) is about 250 µg/mL or less in terms of Compound 1 hydrochloride.
[Item 31] The pharmaceutical preparation according to any one of the items 1 to 28, wherein the concentration of Compound 1, a pharmaceutically acceptable acid addition salt thereof, or a mixture thereof dissolved in the preparation (the dissolution fractional concentration in the preparation) is about 200 µg/mL or less in terms of Compound 1 hydrochloride.
[Item 32] The pharmaceutical preparation according to any one of the items 1 to 28, wherein the concentration of Compound 1, a pharmaceutically acceptable acid addition salt thereof, or a mixture thereof dissolved in the preparation (the dissolution fractional concentration in the preparation) is about 175 µg/mL or less in terms of Compound 1 hydrochloride.
[Item 33] A medicament for treatment and/or prophylaxis of a psychiatric disease, which comprises the pharmaceutical preparation according to any one of the items 1 to 32.
[Item 34] The medicament according to the item 33, wherein the psychiatric disease is schizophrenia, bipolar disorder, senile dementia, or depression.
[Item 35] A method of treating and/or preventing a psychiatric disease, which comprises administering the pharmaceutical preparation according to any one of the items 1 to 32 to a patient in need thereof.
[Item 36] The method according to the item 35, wherein the psychiatric disease is schizophrenia, bipolar disorder, senile dementia, or depression.
[Item 37] Use of the pharmaceutical preparation according to any one of the items 1 to 32 in the manufacture of a medicament for treatment and/or prophylaxis of a psychiatric disease.
[Item 38] The use according to the item 37, wherein the psychiatric disease is schizophrenia, bipolar disorder, senile dementia, or depression.
[Item 39] The pharmaceutical preparation according to any one of the items 1 to 32 for use in the treatment and/or prophylaxis of a psychiatric disease.
[Item 40] The pharmaceutical preparation according to the item 39, wherein the psychiatric disease is schizophrenia, bipolar disorder, senile dementia, or depression.
[Item 41] An agent for controlling dissolution of an aqueous suspension pharmaceutical preparation, comprising one or more chlorides selected from an inorganic chloride or a quaternary ammonium chloride having 4 to 12 carbon atoms.
[Item 42] The agent according to the item 31, wherein the pH is 2.5 - 5.5.
[Item 43] The agent according to the item 41 or 42, wherein the dissolution control is the inhibition of the initial dissolution amount.
[Item 44] An agent for controlling dissolution of an aqueous suspension pharmaceutical preparation with a pH of 2.5 - 5.5 comprising the following (1) and (3):
   (1) (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1), a pharmaceutically acceptable acid addition salt thereof, or a mixture thereof,
   (2) a dispersant, and
   (3) water,
   wherein the agent comprises a chloride.
[Item 45] A method of controlling the dissolution of an aqueous suspension pharmaceutical preparation, which comprises adding a chloride to the aqueous suspension pharmaceutical preparation.
[Item 46] The method according to the item 45, wherein the pH of the aqueous suspension pharmaceutical preparation is 2.5 - 5.5.
[Item 47] The method according to the item 45 or 46, wherein the dissolution control is the inhibition of the initial solubility.

### (EFFECTS OF THE INVENTION)

The present invention does not produce rapid dissolution and absorption even when the aqueous suspension pharmaceutical preparation comprising Compound 1 or salt thereof as an active ingredient is administered orally, and thus can exhibit the solubility in the stomach depending on the property of a drug. As a result, an aqueous suspension pharmaceutical preparation absorbed in gastrointestinal tract with the behavior similar to tablet and capsule can be designed, and also can assure the bioequivalence with the existing tablet. In addition, the present invention can inhibit the particle growth and the aggregation of particles.

### DESCRIPTION OF EMBODIMENTS

Hereinafter, the preferred embodiments of the present invention are explained in detail. However, the present invention is not limited to the following embodiments.

The present invention discloses an aqueous suspension pharmaceutical preparation of Compound 1 or salt thereof suitable for oral administration as a medicament for the treatment of a disease such as schizophrenia.

The term "aqueous suspension pharmaceutical preparation" as used herein comprising Compound 1 or salt thereof as an active ingredient means a preparation in which the suspended particle of Compound 1 or salt thereof is dispersed in a solvent including water as main solvent. The "aqueous suspension pharmaceutical preparation" as used herein may comprise a polar solvent in an amount which produces no negative effect (e.g. bitter taste, astringent taste, astringent) on administration feeling of the preparation besides water.

The term "polar solvent" as used herein means a solvent with polarity which can be homogeneously mixed with water.

(3aR,4S,7R,7aS)-2-{(1R,2R)-2-[4-(1,2-benzisothiazol-3-yl)piperazin-1-ylmethyl]cyclonexylmethyl}hexahydro-4,7-methano-2H-isoindole-1,3-dione (Compound 1) of the present invention is a compound of the following formula:

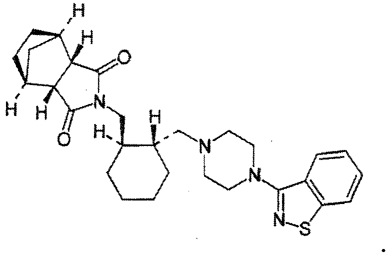

Compound 1 or salt thereof has a pharmacological effect of an antipsychotic agent. More specifically, Compound 1 or salt thereof and a pharmaceutical preparation comprising it are useful as a medicament for treating a psychiatric disease such as schizophrenia, bipolar disorder, and depression.

Examples of the pharmaceutically acceptable acid addition salt of Compound 1 include an organic acid addition salt and an inorganic acid addition salt. Examples of the organic acid addition salt include acetate, lactate, adipate, citrate, tartrate, methanesulfonate, fumarate, and maleate, and examples of the inorganic acid addition salt include hydrochloride, sulfate, nitrate, and phosphate, but are not limited thereto. The pharmaceutically acceptable acid addition salt of Compound 1 is preferably hydrochloride. In addition, Compound 1 and a pharmaceutically acceptable acid addition salt thereof may be in the form of a solvate, a hydrate or a nonhydrate.

Compound 1 or pharmaceutically acceptable acid addition salt thereof can be prepared according to, for example, the processes of Patent Documents 1 and 2 or similar processes thereto. The prepared Compound 1 or pharmaceutically acceptable acid addition salt thereof may be milled according to a commonly-used method as appropriate.

The term "mixture thereof" in "(3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione, a pharmaceutically acceptable acid addition salt thereof, or a mixture thereof" as used herein encompasses (1) a mixture of Compound 1 (in the free form) and a pharmaceutically acceptable acid addition salt of Compound 1 (which may be one or two or more acid addition salts), (2) a mixture of two or more pharmaceutically acceptable acid addition salts of Compound 1.

The content of "(3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione, a pharmaceutically acceptable acid addition salt thereof, or a mixture thereof" as used herein varies depending on the amount of drug solution, but the content thereof in the preparation is basically about 0.1 - about 400 mg/mL in terms of Compound 1 hydrochloride. The content of "Compound 1 or salt thereof" is preferably about 1 - about 300 mg/mL, more preferably about 5 - about 120 mg/mL, furthermore preferably about 10 - about 100 mg/mL, even more preferably about 20 - about 80 mg/mL, particularly preferably about 25 - about 80 mg/mL, and most preferably about 25 - about 50 mg/mL, in terms of Compound 1 hydrochloride. The term "in terms of Compound 1 hydrochloride" as used herein means that the amount of Compound 1, a pharmaceutically acceptable acid addition salt of Compound 1 or a hydrate or solvate thereof is calculated by substituting the amount thereof with the amount of Compound 1 hydrochloride in an equimolar amount thereto.

The term "chloride" as used herein is an inorganic chloride or a quaternary ammonium chloride having 4 to 12 carbon atoms.

Examples of the inorganic chloride include sodium chloride, potassium chloride, iron (II) chloride, calcium chloride, magnesium chloride, zinc chloride, and ammonium chloride. The inorganic chloride is preferably sodium chloride and potassium chloride and more preferably sodium chloride.

The quaternary ammonium chloride having 4 to 12 carbon atoms is not is not particularly limited as long as it is an organic quaternary ammonium chloride in a range of 4 to 12 carbon atoms. The quaternary ammonium chloride is preferably an organic quaternary ammonium chloride with a group such as straight or branched alkyl and alkylene (wherein the quaternary ammonium moiety may have a group such as hydroxy, amino and acyl), and particularly preferably choline chloride.

The concentration of chloride ion in the preparation is about 0.015 - about 1 mol/L, preferably about 0.05 - about 1 mol/L, more preferably about 0.10 - about 1 mol/L, but is not limited thereto. The concentration of chloride ion in the preparation is free of the chloride ion derived from a salt of Compound 1.

The "dispersant" as used herein is not particularly limited as long as it is used as an additive for a conventional suspension preparation. Examples thereof include a cellulose derivative such as cellulose, hypromellose (hydroxypropylmethylcellulose), hydroxypropylcellulose, hydroxyethylcellulose, methylcellulose, ethylcellulose, and carmellose sodium; an alkyl alcohol such as polyvinyl alcohol; a sucrose fatty acid ester such as sucrose myristate, sucrose laurate, and sucrose stearate; a polyglyceryl fatty acid ester such as polyglyceryl myristate, polyglyceryl laurate, and polyglyceryl stearate; a pyrrolidone derivative such as polyvinylpyrrolidone; a non-ionic surfactant such as Poloxamer 188, HCO-60, Polysorbate 80, and Polyoxyl 40 Stearate; an ionic surfactant such as sodium lauryl sulfate. The dispersant is preferably a cellulose derivative, a sucrose fatty acid ester and an alkyl alcohol, more preferably a cellulose derivative, a sucrose fatty acid ester and polyvinyl alcohol, and furthermore preferably a cellulose derivative. Specific examples thereof preferably include hypromellose, hydroxypropylcellulose and hydroxyethyl cellulose, and more preferably hypromellose. The dispersant may also be used alone or in mixture of two or more dispersants.

The content of the dispersant is preferably about 0.01 - about 40 mg/mL of the total amount of the preparation, but is not limited thereto. More specifically, when the dispersant is a cellulose derivative such as hypromellose, hydroxypropylcellulose, hydroxyethyl cellulose or a sucrose fatty acid ester such as sucrose laurate, the content thereof is preferably about 0.1 - about 30 mg/mL, more preferably about 0.1 - about 20 mg/mL, and furthermore preferably about 0.1 - about 10 mg/mL of the total amount of the preparation, but is not limited thereto. Also, when the dispersant is an alkyl alcohol such as polyvinyl alcohol, the content thereof is preferably about 0.1 - about 10 mg/mL, and more preferably about 0.5 - about 5 mg/mL of the total amount of the preparation, but is not limited thereto. When the dispersant is a polyglycerin fatty acid ester, a pyrrolidone derivative, a non-ionic surfactant or an ionic surfactant, the content thereof is preferably about 0.01 - about 0.5 mg/mL, and more preferably about 0.01 - about 0.1 mg/mL of the total amount of the preparation, but is not limited thereto.

The pH of the aqueous suspension pharmaceutical preparation of the present invention is 2.5 - 5.5, preferably 3.0 - 5.5, and more preferably 3.0 - 5.0.

In the aqueous suspension pharmaceutical preparation of the present invention, the dissolution fractional concentration of Compound 1 or salt thereof in the preparation can be measured. The "dissolution fractional concentration in the preparation" of Compound 1 or salt thereof means the concentration of Compound 1 or salt thereof dissolved in the aqueous suspension pharmaceutical preparation. The dissolution fractional concentration in the preparation can be measured by, for example, completely precipitating the suspended particle with a device such as ultracentrifuge and then quantifying the amount of Compound 1 or salt thereof dissolved in the supernatant solution. The amount of the suspended particle of Compound 1 or salt thereof in the aqueous suspension pharmaceutical preparation of the present invention dissolved in 1st fluid for dissolution test described in the Japanese Pharmacopoeia 17th Edition can be measured. The amount can be measured according to, for example, the dissolution test (the paddle method) in the Japanese Pharmacopoeia 17th Edition. In the dissolution test, the preparation comprising 40 mg of Compound 1 or salt thereof in terms of Compound 1 hydrochloride is added into 1st fluid for dissolution test described in the Japanese Pharmacopoeia 17th Edition at 36.5 - 37.5°C, the paddle is rotated in the solution at 50 rpm, the solution is sampled at 10 minutes, 20 minutes and 30 minutes after the start of the test, and the amount of Compound 1 or salt thereof dissolved in the solution is measured.

When the dissolution test (the paddle method) described in the Japanese Pharmacopoeia 17th Edition is performed as described above for the aqueous suspension pharmaceutical preparation of the present invention, the maximum dissolution amount of Compound 1 or salt thereof at 0 - 30 minutes after the start of the test is about 120 µg/mL or less, preferably about 110 µg/mL or less, and more preferably about 100 µg/mL or less in terms of Compound 1 hydrochloride.

The term "agent for controlling dissolution" as used herein means an agent for inhibiting rapid dissolution and absorption of an aqueous suspension pharmaceutical preparation when the preparation is administered orally. The addition of the agent for controlling dissolution of the present invention can produce gastrointestinal absorption with the behavior similar to tablet and capsule and assure the bioequivalence with tablet. Examples of the agent for controlling dissolution of the present invention include a chloride such as an inorganic chloride and a quaternary ammonium chloride having 4 to 12 carbon atoms.

The term "inhibit(s) the initial solubility" means, for example, that the maximum dissolution amount of Compound 1 or salt thereof at 0 to 30 minutes after the start of the above dissolution test is inhibited as compared to the amount thereof prior to the addition of the agent for controlling dissolution. For example, the maximum dissolution amount of Compound 1 or salt thereof at 0 - 30 minutes after the start of the test is about 120 µg/mL or less, preferably about 110 µg/mL or less, and more preferably about 100 µg/mL or less in terms of Compound 1 hydrochloride.

In the aqueous suspension pharmaceutical preparation of the present invention, the concentration of Compound 1 or salt thereof dissolved in the preparation (the dissolution fractional concentration in the preparation) is about 360 µg/mL or less, preferably about 250 µg/mL or less, more preferably about 200 µg/mL or less, and furthermore preferably about 175 µg/mL or less in terms of Compound 1 hydrochloride.

The term "D50" as used herein is one of the representative indexes for representing the particle size distribution of the suspended particle of Compound 1 or salt thereof in the aqueous suspension pharmaceutical preparation and means 50% particle size (median diameter). The D50 of the suspended particle in the aqueous suspension pharmaceutical preparation of the present invention is about 0.1 - about 13 µm, preferably about 0.1 - about 10 µm, and more preferably about 0.1 - about 7 µm, but is not limited thereto.

The term "D90" as used herein means 90% particle size. The D90 of the suspended particle in the aqueous suspension pharmaceutical preparation of the present invention is about 1 - about 30 µm, preferably about 1 - about 20 µm, and more preferably about 1 - about 15 µm, but is not limited thereto.

The suspended particle of Compound 1 or salt thereof whose D50 is about 0.1 - about 13 µm or D90 is about 0.1 - about 30 µm can be produced by dry milling Compound 1 or salt thereof using a mill such as jet mill and hammer mill, but the present invention is not limited thereto. Also, the suspended particle can be produced by adding Compound 1 or salt thereof into a solvent, and then wet-milling the resulting product using a machine such as homomixer, high-speed rotation type disperser, high-pressure homogenizer and bead mill. In addition, the suspended particle can be produced by a commonly-used recrystallization technique of Compound 1 or salt thereof besides the method for milling Compound 1 or salt thereof.

As Compound 1 or salt thereof for using at the time of the manufacture of the aqueous suspension pharmaceutical preparation of the present invention, Compound 1 or salt thereof whose D5C is about 0.1 - about 13 µm or D90 is about 0.1 - about 30 µm produced by an operation such as dry-milling, wet-milling and recrystallization technique can be used, but is not limited thereto. Even if Compound 1 or salt thereof does not have a D50 of about 0.1 - about 13 µm or a D90 of about 0.1 - about 30 µm at the time of the manufacture of the aqueous suspension pharmaceutical preparation of the present invention, the suspended particle of Compound 1 or salt thereof whose D50 is about 0.1 - about 13 µm or D90 is about 0.1 - about 30 µm can be produced by an operation such as wet-milling in the manufacture step.

In general, the particle size is measured with an analyzer such as laser diffraction particle size analyzer, dynamic light scattering particle size analyzer, and image processing particle size analyzer. The D50 and D90 values as used herein can be calculated from the particle size distribution measured with laser diffraction particle size analyzer: HELOS BR-MULTI (Sympatec).

The aqueous suspension pharmaceutical preparation of the present invention may comprise a suspended particle other than the suspended particle of "(3aR,4S,7-R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione, a pharmaceutically acceptable acid addition salt thereof, or a mixture thereof (Compound 1 or salt thereof)" as "suspended particle". Such aqueous suspension pharmaceutical preparation is preferably a preparation wherein the amount of the suspended particle other than that of Compound 1 or salt thereof is an amount which does not affect any property such as the absorbability of such preparation (including a preparation in which the amount is zero), and more preferably a preparation with no suspended particle other than that of Compound 1 or salt thereof.

The aqueous suspension pharmaceutical preparation of the present invention may comprise an additive for a conventional suspension preparation unless the effect of the present invention is lost. Examples of such additive include thickening agent, preservative, stabilizing agent, buffering agent, sweetening agent, coloring agent, and flavoring agent, but are not limited thereto.

The thickening agent is not particularly limited as long as it is used as an additive for a conventional suspension preparation. Examples thereof include polysaccharides such as xanthan gum, guar gum, tamarind gum, gellan gum, carrageenan, carmellose sodium, sodium alginate, pectin, and agar; proteins such as casein and gelatin; acrylic acid polymers such as carboxy vinyl polymer. The thickening agent is preferably polysaccharides and acrylic acid polymers, and more preferably polysaccharides. Specific example thereof preferably include xanthan gum, guar gum, carrageenan, sodium alginate, and pectin, more preferably xanthan gum, guar gum, and sodium alginate, furthermore preferably xanthan gum and sodium alginate. In addition, the thickening agent may be used alone or in a mixture of two or more thickening agents.

The content of the thickening agent is preferably about 0.5 - about 20 mg/mL, more preferably about 1 - about 15 mg/mL, furthermore preferably about 2 - about 10 mg/mL of total amount of the preparation, but is not limited thereto.

The preservative is not particularly limited as long as it is used as an additive for a conventional suspension preparation. Examples thereof include a benzoic acid derivative such as methyl paraoxybenzoate, ethyl paraoxybenzoate, propyl paraoxybenzoate, butyl paraoxybenzoate, paraoxybenzoic acid, benzoic acid, and sodium benzoate; a compound of a backbone comprising three or more carbon atoms with one to four carboxyl groups such as sorbic acid, potassium sorbate, sodium edetate and citric acid; and an alcohol such as glycerin, ethanol, 2-propanol and propylene glycol. The preservative is preferably a benzoic acid derivative. Specific examples thereof preferably include methyl paraoxybenzoate, ethyl paraoxybenzoate, propyl paraoxybenzoate, butyl paraoxybenzoate and sodium benzoate, more preferably methyl paraoxybenzoate, propyl paraoxybenzoate and sodium benzoate, furthermore preferably methyl paraoxybenzoate and sodium benzoate, and most preferably methyl paraoxybenzoate. In addition, the preservative may be used alone or in a mixture of two or more preservatives.

The content of the preservative is preferably about 0.1 - about 10 mg/mL, more preferably about 0.2 - about 5 mg/mL, furthermore preferably about 0.25 - about 3 mg/mL, and particularly preferably about 0.5 - about 2.5 mg/mL of the total amount of the preparation, but is not limited thereto.

The stabilizing agent is not particularly limited as long as it is used as an additive for a conventional suspension preparation. Examples thereof include an alcohol. Examples of the alcohol include a monohydric alcohol such as ethanol; a polyhydric alcohol such as glycerin, propylene glycol, and polyethylene glycol; a sugar alcohol such as sorbitol, erythritol, and mannitol. The stabilizing agent is preferably a polyhydric alcohol. Specific examples thereof preferably include glycerin, sorbitol, erythritol, and propylene glycol, and more preferably propylene glycol. In addition, the stabilizing agent can be used alone or in mixture of two or more stabilizing agents.

The content of the stabilizing agent is preferably about 1 - about 500 mg/mL, more preferably about 10 - about 400 mg/mL, furthermore preferably about 30 - about 350 mg/mL, still furthermore preferably about 50 - about 300 mg/mL, and particularly preferably about 50 - about 150 mg/mL of the total amount of the preparation, but is not limited thereto.

The buffering agent is not particularly limited as long as it is used as an additive for a conventional suspension preparation. Examples thereof include sodium salt, potassium salt, and hydrate thereof. Examples of the sodium salt include disodium phosphate, monosodium phosphate, sodium hydrogen carbonate, sodium carbonate, sodium citrate, and hydrate thereof. Examples of the potassium salt include dipotassium phosphate, monopotassium phosphate, potassium hydrogen carbonate, potassium carbonate, potassium citrate, and hydrate thereof. The buffering agent is preferably sodium salt and potassium salt. Specific examples thereof preferably include dipotassium phosphate, monopotassium phosphate, disodium phosphate, monosodium phosphate, and hydrate thereof, more preferably dipotassium phosphate, monopotassium phosphate, disodium phosphate, monosodium phosphate, and hydrate thereof, furthermore preferably dipotassium phosphate, monosodium phosphate, and hydrate thereof. In addition, the buffering agent may be used alone or in a mixture of two or more buffering agents.

Dipotassium phosphate is the same compound as dipotassium hydrogen phosphate, and monopotassium phosphate is the same compound as potassium dihydrogen phosphate.

The content of the buffering agent in the aqueous suspension pharmaceutical preparation of the present invention is preferably about 0 - about 15 mg, more preferably about 0 - about 5 mg, furthermore preferably about 0 - about 0.85 mg, and particularly preferably about 0 - about 0.5 mg relative to 1 mg of Compound 1 or salt thereof in the preparation (the weight in terms of Compound 1 hydrochloride), but is not limited thereto. When the content of the buffering agent exceeds 15 mg relative to 1 mg of Compound 1 or salt thereof in the preparation (the weight in terms of Compound 1 hydrochloride), there is the possibility to make the maintenance of the suspended state difficult because of the deposition of other additive such as a dispersant by salting out.

The sweetening agent is not particularly limited as long as it is used as an additive for a conventional suspension preparation. Examples thereof include sucralose, stevia, sucrose, liquid sugar, fructose, sorbitol, xylitol, erythritol, trehalose, maltitol, and acesulfame potassium. The sweetening agent is preferably sucralose, stevia, sorbitol, and erythritol, more preferably sucralose, stevia, and erythritol, and furthermore preferably sucralose and stevia. In addition, the sweetening agent may be used alone or in mixture of two or more sweetening agents.

The coloring agent is not particularly limited as long as it is used as an additive for a conventional suspension preparation. Examples thereof include tar dye, Yellow No.5, and caramel. In addition, the coloring agent may be used alone or in a mixture of two or more coloring agents.

The flavoring agent is not particularly limited as long as it is used as an additive for a conventional suspension preparation. Examples thereof include apple flavor, medical essence, lemon oil, orange oil, and peach oil. In addition, the flavoring agent may be used alone or in a mixture of two or more flavoring agents.

The aqueous suspension pharmaceutical preparation of the present invention can be prepared by the preparation process of a conventional oral solution. For example, the aqueous suspension pharmaceutical preparation of the present invention can be prepared by the process comprising the following steps (1)-(5), but is not limited thereto.
(1) A dispersant and other additive are added to a solvent and dissolved.
(2) To the solution prepared in the above (1) is added the dry-milled Compound 1 or salt thereof and mixed to prepare Solution A.
(3) As appropriate, the clump of Compound 1 or salt thereof added in the above (2) is loosened with a machine such as homomixer, stirring machine and high-speed rotation type disperser (wet-milling) or the clump is stirred or dispersed to homogeneously suspend Compound 1 or salt thereof.
(4) Separately, in another container, an additive other than a dispersant is added to a solvent and dissolved to prepare Solution B.
(5) The aqueous suspension pharmaceutical preparation of the present invention can be prepared by mixing Solution A and Solution B prepared in the above in an appropriate amount.

In the process comprising the above (1) - (5), it is preferable that Compound 1 or salt thereof and a dispersant are contained in Solution A, and a thickening agent is contained in Solution B. On the other hand, other additives may be contained in Solution A, Solution B or both of Solution A and Solution B.

When an additive is added to a solvent and dissolved in the manufacture of the aqueous suspension pharmaceutical preparation of the present invention, the additive can also be dissolved with a heated solvent. Also, the additive is preliminarily dissolved in a solvent such as propylene glycol, ethanol and glycerin and the resulting solution may be mixed to a solvent. These processes are helpful when an additive with low solubility in a solvent is dissolved to the solvent. Examples of the additive with low solubility in a solvent include preservative, but are not limited thereto.

The aqueous suspension pharmaceutical preparation of the present invention have no specific disadvantage even if the preparation has air bubbles. The preparation process may be modified so that the preparation has no air bubble. Specifically, the aqueous suspension pharmaceutical preparation of the present invention in which no air bubble is visually contained can be prepared, for example, by appropriately modifying any preparation condition such as processing time, processing strength, liquid temperature of processed product and indoor pressure in the wet-milling, stirring or dispersion step, but the present invention is not limited thereto. Even if air bubbles are contained in the preparation in the preparation process, the contained air bubbles can be removed from the aqueous suspension pharmaceutical preparation of the present invention by the introduction of a step such as reduced pressure step and stirring step.

The storage container of the aqueous suspension pharmaceutical preparation of the present invention is not particularly limited as long as it is a storage container for a conventional oral solution or injection medicine. Examples thereof include a vial, an ample, a glass bottle, a polyethylene bottle and a container divided by aluminum multilayer film.

The aqueous suspension pharmaceutical preparation of the present invention can be prepared in an appropriate concentration depending on factors such as dosage, and administered by adjusting the dosage as appropriate.

Even if the aqueous suspension pharmaceutical preparation of the present invention is administered orally, the preparation has little effect on gastrointestinal tract motility and environment in the gastrointestinal tract. As a result, the aqueous suspension pharmaceutical preparation of the present invention can also be administered in emergency situations.

### EXAMPLES

Hereinafter, the present invention is explained in more detail in Examples, Comparative Examples, Test Examples, but should not be limited thereto.

In the following Examples and Comparative Examples, the following hypromellose, sodium chloride, propylene glycol, sodium benzoate, methylparaben, xanthan gum, dipotassium phosphate, monopotassium phosphate and sucralose were used, but the present invention is not limited thereto.
- hypromellose: TC-5R (Shin-Etsu Chemical Co., Ltd.)
- sodium chloride: Sodium chloride suitable for the biopharmaceutical production (Merck Co., Ltd.)
- propylene glycol: Japanese Pharmacopoeia propylene glycol (ADEKA)
- sodium benzoate: Japanese Pharmacopoeia sodium benzoate (Fushimi Pharmaceutical Co., Ltd.)
- methylparaben: sodium paraoxybenzoate (Ueno Fine Chemicals Industry. Ltd.)
- xanthan gum: Echogum T/KELTROL T (DSP GOKYO FOOD & CHEMICAL Co., Ltd.)
- dipotassium phosphate: dipotassium hydrogen phosphate (Taihei Chemical Industrial Co., Ltd.)
- monopotassium phosphate: potassium dihydrogen phosphate (Taihei Chemical Industrial Co., Ltd.)
- sucralose: Sucralose P (San-Ei Gen F.F.I, Inc.)

### Examples 1-5

According to the following procedure, the preparations of Examples 1-5 were prepared from Compound 1 hydrochloride, an additive and a solvent in the amounts shown in Table below.

### (1) Preparation of Solution A

Hypromellose, sodium benzoate and propylene glycol were added to purified water and dissolved. Sodium chloride, potassium chloride, magnesium chloride hexahydrate, and iron (II) chloride tetrahydrate or calcium chloride was then added thereto and dissolved. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

### (2) Preparation of Solution B

Sodium benzoate and propylene glycol were added to purified water and dissolved. Sodium chloride, potassium chloride, magnesium chloride hexahydrate, and iron (II) chloride tetrahydrate or calcium chloride was then added thereto and dissolved. The solution was then warmed to 70°C, and xanthan gum was added thereto and dissolved. The solution was then cooled to room temperature to prepare Solution B.

### (3) Preparation of Examples 1-5

Solution A and Solution B were mixed in a mass ratio of 1:1 to prepare the preparations of Examples 1-5.

### (4) pH measurement of Examples 1-5

The pH of each preparation of Examples 1-5 was measured according to the method described in the general test method in the Japanese Pharmacopoeia 17th Edition.

**[Table 1]**

| Formulation amount (Content) | Example 1 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 1.0 g (10 mg/mL) |
| sodium chloride | 1.0 g (10 mg/mL) | 1.0 g (10 mg/mL) |
| purified water | 100 mL in total | 100 mL in total |

**[Table 2]**

| Formulation amount (Content) | Example 2 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 1.0 g (10 mg/mL) |
| potassium chloride | 1.0 g (10 mg/mL) | 1.0 g (10 mg/mL) |
| purified water | 100 mL in total | 100 mL in total |

**[Table 3]**

| Formulation amount (Content) | Example 3 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 1.0 g (10 mg/mL) |
| magnesium chloride hexahydrate | 1.0 g (10 mg/mL) | 1.0 g (10 mg/mL) |
| purified water | 100 mL in total | 100 mL in total |

**[Table 4]**

| Formulation amount (Content) | Example 4 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 1.0 g (10 mg/mL) |
| iron (II) chloride tetrahydrate | 1.0 g (10 mg/mL) | 1.0 g (10 mg/mL) |
| purified water | 100 mL in total | 100 mL in total |

**[Table 5]**

| Formulation amount (Content) | Example 5 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 1.0 g (10 mg/mL) |
| calcium chloride | 1.0 g (10 mg/mL) | 1.0 g (10 mg/mL) |
| purified water | 100 mL in total | 100 mL in total |

The formulation amount and content of each ingredient in the preparations of the above Examples are shown in Table below.

**[Table 6]**

| Formulation amount (Content) | Example 1 | Example 2 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| sodium benzoate | 0.4 g (2.0 mg/mL) | 0.4 g (2.0 mg/mL) |
| xanthan gum | 1.0 g (5.0 mg/mL) | 1.0 g (5.0 mg/mL) |
| sodium chloride | 2.0 g (10 mg/mL) | - |
| potassium chloride | - | 2.0 g (10 mg/mL) |
| purified water | 200 mL in total | 200 mL in total |
| pH | 4.33 | 4.30 |

**[Table 7]**

| Formulation amount (Content) | Example 3 | Example 4 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| sodium benzoate | 0.4 g (2.0 mg/mL) | 0.4 g (2.0 mg/mL) |
| xanthan gum | 1.0 g (5.0 mg/mL) | 1.0 g (5.0 mg/mL) |
| magnesium chloride hexahydrate | 2.0 g (10 mg/mL) | - |
| iron (II) chloride tetrahydrate | - | 2.0 g (10 mg/mL) |
| purified water | 200 mL in total | 200 mL in total |
| pH | 4.13 | 3.80 |

**[Table 8]**

| Formulation amount (Content) | Example 5 |
|---|---|
| Compound 1 hydrochloride | 8.0 g (40 mg/mL) |
| hypromellose | 2.0 g (10 mg/mL) |
| propylene glycol | 10.0 g (50 mg/mL) |
| sodium benzoate | 0.4 g (2.0 mg/mL) |
| xanthan gum | 1.0 g (5.0 mg/mL) |
| calcium chloride | 2.0 g (10 mg/mL) |
| purified water | 200 mL in total |
| pH | 4.23 |

### Examples 6-13

According to the following procedure, the preparations of Examples 6-13 were prepared from Compound 1 hydrochloride, an additive and a solvent in the amounts shown in Table below.

### (1) Preparation of Solution A

Methylparaben or sodium benzoate was added to purified water at about 70°C and dissolved, and then the solution was cooled to room temperature. Hypromellose, sucralose, and propylene glycol were then added thereto and dissolved. Sodium chloride or choline chloride was then added thereto and dissolved. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

### (2) Preparation of Solution B

Methylparaben or sodium benzoate was added to purified water at about 70°C and dissolved, and then propylene glycol was added thereto and dissolved. Sodium chloride or choline chloride was then added thereto and dissolved. Xanthan gum was then added thereto and dissolved. The solution was then cooled to room temperature to prepare Solution B.

### (3) Preparation of Examples 6-13

Solution A and Solution B were mixed in a mass ratio of 1:1 to prepare the preparations of Examples 1-5.

### (4) pH measurement of Examples 6-13

The pH of each preparation of Examples 6-13 was measured according to the method described in the general test method in the Japanese Pharmacopoeia 17th Edition.

**[Table 9]**

| Formulation amount (Content) | Example 6 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| methylparaben | 0.2 g (2.0 mg/mL) | 0.2 g (2.0 mg/mL) |
| sucralose | 0.2 g (2.0 mg/mL) | - |
| xanthan gum | - | 1.2 g (12 mg/mL) |
| sodium chloride | 0.5 g (5.0 mg/mL) | 0.5 g (5.0 mg/mL) |
| purified water | 100 mL in total | 100 mL in total |

**[Table 10]**

| Formulation amount (Content) | Example 7 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| methylparaben | 0.2 g (2.0 mg/mL) | 0.2 g (2.0 mg/mL) |
| sucralose | 0.2 g (2.0 mg/mL) | - |
| xanthan gum | - | 1.2 g (12 mg/mL) |
| sodium chloride | 1.0 g (10 mg/mL) | 1.0 g (10 mg/mL) |
| purified water | 100 mL in total | 100 mL in total |

**[Table 11]**

| Formulation amount (Content) | Example 8 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| methylparaben | 0.2 g (2.0 mg/mL) | 0.2 g (2.0 mg/mL) |
| sucralose | 0.2 g (2.0 mg/mL) | - |
| xanthan gum | - | 1.2 g (12 mg/mL) |
| sodium chloride | 2.0 g (20 mg/mL) | 2.0 g (20 mg/mL) |
| purified water | 100 mL in total | 100 mL in total |

**[Table 12]**

| Formulation amount (Content) | Example 9 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| methylparaben | 0.2 g (2.0 mg/mL) | 0.2 g (2.0 mg/mL) |
| sucralose | 0.2 g (2.0 mg/mL) | - |
| xanthan gum | - | 1.2 g (12 mg/mL) |
| choline chloride | 2.0 g (20 mg/mL) | 2.0 g (20 mg/mL) |
| purified water | 100 mL in total | 100 mL in total |

**[Table 13]**

| Formulation amount (Content) | Example 10 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| methylparaben | 0.2 g (2.0 mg/mL) | 0.2 g (2.0 mg/mL) |
| sucralose | 0.2 g (2.0 mg/mL) | - |
| xanthan gum | - | 1.2 g (12 mg/mL) |
| choline chloride | 4.0 g (40 mg/mL) | 4.0 g (40 mg/mL) |
| purified water | 100 mL in total | 100 mL in total |

**[Table 14]**

| Formulation amount (Content) | Example 11 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| methylparaben | 0.2 g (2.0 mg/mL) | 0.2 g (2.0 mg/mL) |
| sucralose | 0.2 g (2.0 mg/mL) | - |
| xanthan gum | - | 1.2 g (12 mg/mL) |
| choline chloride | 6.0 g (60 mg/mL) | 6.0 g (60 mg/mL) |
| purified water | 100 mL in total | 100 mL in total |

**[Table 15]**

| Formulation amount (Content) | Example 12 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| sucralose | 0.2 g (2.0 mg/mL) | - |
| xanthan gum | - | 1.2 g (12 mg/mL) |
| sodium chloride | 1.0 g (10 mg/mL) | 1.0 g (10 mg/mL) |
| purified water | 100 mL in total | 100 mL in total |

**[Table 16]**

| Formulation amount (Content) | Example 13 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| sucralose | 0.2 g (2.0 mg/mL) | - |
| xanthan gum | - | 1.2 g (12 mg/mL) |
| choline chloride | 4.0 g (40 mg/mL) | 4.0 g (40 mg/mL) |
| purified water | 100 mL in total | 100 mL in total |

The formulation amount and content of each ingredient in the preparations of the above Examples are shown in Table below.

**[Table 17]**

| Formulation amount (Content) | Example 6 | Example 7 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| methylparaben | 0.4 g (2.0 mg/mL) | 0.4 g (2.0 mg/mL) |
| sucralose | 0.2 g (1.0 mg/mL) | 0.2 g (1.0 mg/mL) |
| xanthan gum | 1.2 g (6.0 mg/mL) | 1.0 g (5.0 mg/mL) |
| sodium chloride | 1.0 g (5.0 mg/mL) | 2.0 g (10 mg/mL) |
| purified water | 200 mL in total | 200 mL in total |
| pH | 4.23 | 4.46 |

**[Table 18]**

| Formulation amount (Content) | Example 8 | Example 9 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| methylparaben | 0.4 g (2.0 mg/mL) | 0.4 g (2.0 mg/mL) |
| sucralose | 0.2 g (1.0 mg/mL) | 0.2 g (1.0 mg/mL) |
| xanthan gum | 1.2 g (6.0 mg/mL) | 1.0 g (5.0 mg/mL) |
| sodium chloride | 4.0 g (20 mg/mL) | - |
| choline chloride | - | 4.0 g (20 mg/mL) |
| purified water | 200 mL in total | 200 mL in total |
| pH | 4.70 | 4.42 |

**[Table 19]**

| Formulation amount (Content) | Example 10 | Example 11 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| methylparaben | 0.4 g (2.0 mg/mL) | 0.4 g (2.0 mg/mL) |
| sucralose | 0.2 g (1.0 mg/mL) | 0.2 g (1.0 mg/mL) |
| xanthan gum | 1.2 g (6.0 mg/mL) | 1.0 g (5.0 mg/mL) |
| choline chloride | 8.0 g (40 mg/mL) | 12.0 g (60 mg/mL) |
| purified water | 200 mL in total | 200 mL in total |
| pH | 4.60 | 4.75 |

**[Table 20]**

| Formulation amount (Content) | Example 12 | Example 13 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| sodium benzoate | 0.4 g (2.0 mg/mL) | 0.4 g (2.0 mg/mL) |
| sucralose | 0.2 g (1.0 mg/mL) | 0.2 g (1.0 mg/mL) |
| xanthan gum | 1.2 g (6.0 mg/mL) | 1.0 g (5.0 mg/mL) |
| sodium chloride | 2.0 g (10 mg/mL) | - |
| choline chloride | - | 8.0 g (40 mg/mL) |
| purified water | 200 mL in total | 200 mL in total |
| pH | 4.58 | 4.75 |

### Examples 14 and 15

According to the process of Examples 1-5, the preparations of Examples 14 and 15 were prepared from Compound 1 hydrochloride, an additive and a solvent in the amounts shown in Table below.

**[Table 21]**

| Formulation amount (Content) | Example 14 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 1.0 g (10 mg/mL) |
| sodium chloride | 0.1 g (1.0 mg/mL) | 0.1 g (1.0 mg/mL) |
| purified water | 100 mL in total | 100 mL in total |

**[Table 22]**

| Formulation amount (Content) | Example 15 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 1.0 g (10 mg/mL) |
| sodium chloride | 0.3 g (3.0 mg/mL) | 0.3 g (3.0 mg/mL) |
| purified water | 100 mL in total | 100 mL in total |

The formulation amount and content of each ingredient in the preparations of the above Examples are shown in Table below.

**[Table 23]**

| Formulation amount (Content) | Example 14 | Example 15 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| sodium benzoate | 0.4 g (2.0 mg/mL) | 0.4 g (2.0 mg/mL) |
| xanthan gum | 1.0 g (5.0 mg/mL) | 1.0 g (5.0 mg/mL) |
| sodium chloride | 0.2 g (1.0 mg/mL) | 0.6 g (3.0 mg/mL) |
| purified water | 200 mL in total | 200 mL in total |
| pH | 3.80 | 4.11 |

### Examples 16-19

According to the following procedure, the preparations of Examples 16-19 were prepared from Compound 1 hydrochloride, an additive and a solvent in the amounts shown in Table below.

### (1) Preparation of Solution A

Methylparaben was added to purified water at about 70°C and dissolved, and then the solution was cooled to room temperature. Hypromellose, dipotassium phosphate, propylene glycol, and sodium chloride were then added thereto and dissolved. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

### (2) Preparation of Solution B

Methylparaben was added to purified water at about 70°C and dissolved, and then propylene glycol and sodium chloride were added thereto and dissolved. Xanthan gum was then added thereto and dissolved. The solution was then cooled to room temperature to prepare Solution B.

### (3) Preparation of Examples 16-19

Solution A and Solution B were mixed in a mass ratio of 1:1 to prepare the preparations of Examples 16-19.

### (4) pH measurement of Examples 16-19

The pH of each preparation of Examples 16-19 was measured according to the method described in the general test method in the Japanese Pharmacopoeia 17th Edition.

**[Table 24]**

| Formulation amount (Content) | Example 16 | |
|---|---|---|
| | Solution A | Solution B |
| Compound 1 hydrochloride | 8.0 g (80 mg/mL) | - |
| hypromellose | 3.0 g (30 mg/mL) | - |
| propylene glycol | 5.0 g (50 mg/mL) | 5.0 g (50 mg/mL) |
| methylparaben | 0.2 g (2.0 mg/mL) | 0.2 g (2.0 mg/mL) |
| xanthan gum | - | 1.0 g (10 mg/mL) |
| sodium chloride | 1.0 g (10 mg/mL) | 1.0 g (10 mg/mL) |
| dipotassium phosphate | 0.05 g(0.5 mg/mL) | - |
| purified water | 100 mL in total | 100 mL in total |

**[Table 25]**

| Formulation amount (Content) | Example 17 | |
|---|---|---|
| | Solution A | Solution B |
| Compound 1 hydrochloride | 8.0 g (80 mg/mL) | - |
| hypromellose | 3.0 g (30 mg/mL) | - |
| propylene glycol | 5.0 g (50 mg/mL) | 5.0 g (50 mg/mL) |
| methylparaben | 0.2 g (2.0 mg/mL) | 0.2 g (2.0 mg/mL) |
| xanthan gum | - | 1.0 g (10 mg/mL) |
| sodium chloride | 1.0 g (10 mg/mL) | 1.0 g (10 mg/mL) |
| dipotassium phosphate | 0.1 g (1.0 mg/mL) | - |
| purified water | 100 mL in total | 100 mL in total |

**[Table 26]**

| Formulation amount (Content) | Example 18 | |
|---|---|---|
| | Solution A | Solution B |
| Compound 1 hydrochloride | 8.0 g (80 mg/mL) | - |
| hypromellose | 3.0 g (30 mg/mL) | - |
| propylene glycol | 5.0 g (50 mg/mL) | 5.0 g (50 mg/mL) |
| methylparaben | 0.2 g (2.0 mg/mL) | 0.2 g (2.0 mg/mL) |
| xanthan gum | - | 1.0 g (10 mg/mL) |
| sodium chloride | 1.0 g (10 mg/mL) | 1.0 g (10 mg/mL) |
| dipotassium phosphate | 0.2 g (2.0 mg/mL) | - |
| purified water | 100 mL in total | 100 mL in total |

**[Table 27]**

| Formulation amount (Content) | Example 19 | |
|---|---|---|
| | Solution A | Solution B |
| Compound 1 hydrochloride | 8.0 g (80 mg/mL) | - |
| hypromellose | 3.0 g (30 mg/mL) | - |
| propylene glycol | 5.0 g (50 mg/mL) | 5.0 g (50 mg/mL) |
| methylparaben | 0.2 g (2.0 mg/mL) | 0.2 g (2.0 mg/mL) |
| xanthan gum | - | 1.0 g (10 mg/mL) |
| sodium chloride | 1.0 g (10 mg/mL) | 1.0 g (10 mg/mL) |
| dipotassium phosphate | 0.3 g (3.0 mg/mL) | - |
| purified water | 100 mL in total | 100 mL in total |

The formulation amount and content of each ingredient in the preparations of the above Examples are shown in Table below.

**[Table 28]**

| Formulation amount (Content) | Example 16 | Example 17 |
|---|---|---|
| Compound 1 hydrochloride | 8.0 g (40 mg/mL) | 8.0 g (40 mg/mL) |
| hypromellose | 3.0 g (15 mg/mL) | 3.0 g (15 mg/mL) |
| propylene glycol | 10.0 g (50 mg/mL) | 10.0 g (50 mg/mL) |
| methylparaben | 0.4 g (2.0 mg/mL) | 0.4 g (2.0 mg/mL) |
| xanthan gum | 1.0 g (5.0 mg/mL) | 1.0 g (5.0 mg/mL) |
| sodium chloride | 2.0 g (10 mg/mL) | 2 g (10 mg/mL) |
| dipotassium phosphate | 0.05 g (0.25 mg/mL) | 0.1 g (0.5 mg/mL) |
| purified water | 200 mL in total | 200 mL in total |
| pH | 4.48 | 4.48 |

**[Table 29]**

| Formulation amount (Content) | Example 18 | Example 19 |
|---|---|---|
| Compound 1 hydrochloride | 8.0 g (40 mg/mL) | 8.0 g (40 mg/mL) |
| hypromellose | 3.0 g (15 mg/mL) | 3.0 g (15 mg/mL) |
| propylene glycol | 10.0 g (50 mg/mL) | 10.0 g (50 mg/mL) |
| methylparaben | 0.4 g (2.0 mg/mL) | 0.4 g (2.0 mg/mL) |
| xanthan gum | 1.0 g (5.0 mg/mL) | 1.0 g (5.0 mg/mL) |
| sodium chloride | 2.0 g (10 mg/mL) | 2 g (10 mg/mL) |
| dipotassium phosphate | 0.2 g (1.0 mg/mL) | 0.3 g (1.5 mg/mL) |
| purified water | 200 mL in total | 200 mL in total |
| pH | 4.49 | 4.51 |

### Examples 20-23

According to the following procedure, the preparations of Examples 20-23 were prepared from Compound 1 hydrochloride, an additive and a solvent in the amounts shown in Table below.

### (1) Preparation of Solution A

Hypromellose, sodium benzoate, sodium chloride, sucralose, and propylene glycol were added to purified water and dissolved. In Example 20, stevia and apple flavor were further added thereto and dissolved; in Example 21, stevia, apple flavor and erythritol were further added thereto and dissolved; in Example 22, neotame and vanillin were further added thereto and dissolved. To each solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

### (2) Preparation of Solution B

Sodium benzoate, sodium chloride, and propylene glycol were added to purified water and dissolved. Erythritol was then added thereto and dissolved. The solution was then warmed to 70°C, and xanthan gum was added thereto and dissolved. The solution was then cooled to room temperature to prepare Solution B.

### (3) Preparation of Examples 20-23

Solution A and Solution B were mixed in a mass ratio of 1:1 to prepare the preparations of Examples 20-23.

### (4) pH measurement of Examples 20-23

The pH of each preparation of Examples 20-23 was measured according to the method described in the general test method in the Japanese Pharmacopoeia 17th Edition.

**[Table 30]**

| Formulation amount (Content) | Example 20 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 1.0 g (10 mg/mL) |
| sodium chloride | 1.0 g (10 mg/mL) | 1.0 g (10 mg/mL) |
| sucralose | 0.2 g (2.0 mg/mL) | - |
| stevia | 0.2 g (2.0 mg/mL) | - |
| apple flavor | 0.04 g (0.4 mg/mL) | - |
| purified water | 100 mL in total | 100 mL in total |

**[Table 31]**

| Formulation amount (Content) | Example 21 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 1.6 g (16 mg/mL) |
| sodium chloride | 1.0 g (10 mg/mL) | 1.0 g (10 mg/mL) |
| sucralose | 0.2 g (2.0 mg/mL) | - |
| stevia | 0.2 g (2.0 mg/mL) | - |
| apple flavor | 0.04 g (0.4 mg/mL) | - |
| erythritol | 10 g (100 mg/mL) | 10 g (100 mg/mL) |
| purified water | 100 mL in total | 100 mL in total |

**[Table 32]**

| Formulation amount (Content) | Example 22 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 1.0 g (10 mg/mL) |
| sodium chloride | 1.0 g (10 mg/mL) | 1.0 g (10 mg/mL) |
| sucralose | 0.2 g (2.0 mg/mL) | - |
| neotame | 0.002 g (0.02 mg/mL) | - |
| vanillin | 0.2 g (2.0 mg/mL) | - |
| purified water | 100 mL in total | 100 mL in total |

**[Table 33]**

| Formulation amount (Content) | Example 23 | |
|---|---|---|
| | Solution A | Solution B |
| Compound 1 hydrochloride | 4.0 g (40 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 1.0 g (10 mg/mL) |
| sodium chloride | 1.0 g (10 mg/mL) | 1.0 g (10 mg/mL) |
| sucralose | 0.2 g (2.0 mg/mL) | - |
| neotame | 0.002 g (0.02 mg/mL) | - |
| vanillin | 0.2 g (2.0 mg/mL) | - |
| purified water | 100 mL in total | 100 mL in total |

The formulation amount and content of each ingredient in the preparations of the above Examples are shown in Table below.

**[Table 34]**

| Formulation amount (Content) | Example 20 | Example 21 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| sodium benzoate | 0.4 g (2.0 mg/mL) | 0.4 g (2.0 mg/mL) |
| xanthan gum | 1.0 g (5 mg/mL) | 1.6 g (8 mg/mL) |
| sodium chloride | 2.0 g (10 mg/mL) | 2.0 g (10 mg/mL) |
| sucralose | 0.2 g (1.0 mg/mL) | 0.2 g (1.0 mg/mL) |
| stevia | 0.2 g (1.0 mg/mL) | 0.2 g (1.0 mg/mL) |
| apple flavor | 0.04 g (0.2 mg/mL) | 0.04 g (0.2 mg/mL) |
| erythritol | - | 20 g (200 mg/mL) |
| purified water | 200 mL in total | 200 mL in total |
| pH | 4.52 | 4.52 |

**[Table 35]**

| Formulation amount (Content) | Example 22 | Example 23 |
|---|---|---|
| Compound 1 hydrochloride | 8.0 g (40 mg/mL) | 4.0 g (20 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| sodium benzoate | 0.4 g (2.0 mg/mL) | 0.4 g (2.0 mg/mL) |
| xanthan gum | 1.0 g (5.0 mg/mL) | 1.0 g (5.0 mg/mL) |
| sodium chloride | 2.0 g (10 mg/mL) | 2.0 g (10 mg/mL) |
| sucralose | 0.2 g (1. 0 mg/mL) | 0.2 g (1.0 mg/mL) |
| neotame | 0.002 g (0.01 mg/mL) | 0.002 g (0.01 mg/mL) |
| vanillin | 0.2 g (1.0 mg/mL) | 0.2 g (1.0 mg/mL) |
| purified water | 200 mL in total | 200 mL in total |
| pH | 4.50 | 4.53 |

### Examples 24-26

According to the following procedure, the preparations of Examples 24-26 were prepared from Compound 1 hydrochloride, an additive and a solvent in the amounts shown in Table below.

### (1) Preparation of Solution A

Hypromellose, sodium benzoate, sodium chloride, and sucralose were added to purified water and dissolved. Stevia, erythritol, sodium alginate, and apple flavor were then added thereto and dissolved. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

### (2) Preparation of Solution B

Sodium benzoate and propylene glycol were added to purified water and dissolved. The solution was then warmed to 70°C, and xanthan gum was added thereto and dissolved. The solution was then cooled to room temperature to prepare Solution B.

### (3) Preparation of Examples 24-26

Solution A and Solution B were mixed in a mass ratio of 1:1 to prepare the preparations of Examples 24-26.

### (4) pH measurement of Examples 24-26

The pH of each preparation of Examples 24-26 was measured according to the method described in the general test method in the Japanese Pharmacopoeia 17th Edition.

**[Table 36]**

| Formulation amount (Content) | Example 24 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | - | 10.0 g (100 mg/mL) |
| sodium benzoate | 0.2 g (2.0 mg/mL) | 0.2 g (2.0 mg/mL) |
| xanthan gum | - | 1.6 g (16 mg/mL) |
| sodium chloride | 2.0 g (20 mg/mL) | - |
| sodium alginate | 0.04 g (0.4 mg/mL) | - |
| sucralose | 0.2 g (2.0 mg/mL) | - |
| stevia | 0.2 g (2.0 mg/mL) | - |
| apple flavor | 0.04 g (0.4 mg/mL) | - |
| erythritol | 10.0 g (100 mg/mL) | - |
| purified water | 100 mL in total | 100 mL in total |

**[Table 37]**

| Formulation amount (Content) | Example 25 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | - | 10.0 g (100 mg/mL) |
| sodium benzoate | 0.2 g (2.0 mg/mL) | 0.2 g (2.0 mg/mL) |
| xanthan gum | - | 1.0 g (10 mg/mL) |
| sodium chloride | 2.0 g (20 mg/mL) | - |
| sodium alginate | 0.2 g (2.0 mg/mL) | - |
| sucralose | 0.2 g (2.0 mg/mL) | - |
| stevia | 0.2 g (2.0 mg/mL) | - |
| apple flavor | 0.04 g (0.4 mg/mL) | - |
| erythritol | 10.0 g (100 mg/mL) | - |
| purified water | 100 mL in total | 100 mL in total |

**[Table 38]**

| Formulation amount (Content) | Example 26 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | - | 10.0 g (100 mg/mL) |
| sodium benzoate | 0.2 g (2.0 mg/mL) | 0.2 g (2.0 mg/mL) |
| xanthan gum | - | 1.6 g (16 mg/mL) |
| sodium chloride | 2.0 g (20 mg/mL) | - |
| sodium alginate | 0.08 g (0.8 mg/mL) | - |
| sucralose | 0.2 g (2.0 mg/mL) | - |
| stevia | 0.2 g (2.0 mg/mL) | - |
| apple flavor | 0.04 g (0.4 mg/mL) | - |
| erythritol | 10.0 g (100 mg/mL) | - |
| purified water | 100 mL in total | 100 mL in total |

The formulation amount and content of each ingredient in the preparations of the above Examples are shown in Table below.

**[Table 39]**

| Formulation amount (Content) | Example 24 | Example 25 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| sodium benzoate | 0.4 g (2.0 mg/mL) | 0.4 g (2.0 mg/mL) |
| xanthan gum | 1.6 g (8 mg/mL) | 1.0 g (5 mg/mL) |
| sodium chloride | 2.0 g (10 mg/mL) | 2.0 g (10 mg/mL) |
| sodium alginate | 0.04 g (0.2 mg/mL) | 0.2 g (1.0 mg/mL) |
| sucralose | 0.2 g (1.0 mg/mL) | 0.2 g (1.0 mg/mL) |
| stevia | 0.2 g (1.0 mg/mL) | 0.2 g (1.0 mg/mL) |
| apple flavor | 0.04 g (0.2 mg/mL) | 0.04 g (0.2 mg/mL) |
| erythritol | 10.0 g (50 mg/mL) | 10.0 g (50 mg/mL) |
| purified water | 200 mL in total | 200 mL in total |
| pH | 4.58 | 4.50 |

**[Table 40]**

| Formulation amount (Content) | Example 26 |
|---|---|
| Compound 1 hydrochloride | 8.0 g (40 mg/mL) |
| hypromellose | 2.0 g (10 mg/mL) |
| propylene glycol | 10.0 g (50 mg/mL) |
| sodium benzoate | 0.4 g (2.0 mg/mL) |
| xanthan gum | 1.6 g (8 mg/mL) |
| sodium chloride | 2.0 g (10 mg/mL) |
| sodium alginate | 0.08 g (0.4 mg/mL) |
| sucralose | 0.2 g (1.0 mg/mL) |
| stevia | 0.2 g (1.0 mg/mL) |
| apple flavor | 0.04 g (0.2 mg/mL) |
| erythritol | 10.0 g (50 mg/mL) |
| purified water | 200 mL in total |
| pH | 4.49 |

### Examples 27-29

According to the following procedure, the preparations of Examples 27-29 were prepared from Compound 1 hydrochloride, an additive and a solvent in the amounts shown in Table below.

### (1) Preparation of Solution A

Hypromellose, sodium benzoate, sodium chloride, and sucralose were added to purified water and dissolved. Stevia and apple flavor were then added thereto and dissolved. In Example 27, erythritol was further added thereto and dissolved. To each solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

### (2) Preparation of Solution B

Sodium benzoate and propylene glycol were added to purified water and dissolved. The solution was then warmed to 70°C, and xanthan gum was added thereto and dissolved. The solution was then cooled to room temperature to prepare Solution B.

### (3) Preparation of Examples 27-29

Solution A and Solution B were mixed in a mass ratio of 1:1 to prepare the preparations of Examples 27-29.

### (4) pH measurement of Examples 27-29

The pH of each preparation of Examples 27-29 was measured according to the method described in the general test method in the Japanese Pharmacopoeia 17th Edition.

**[Table 41]**

| Formulation amount (Content) | Example 27 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | - | 10.0 g (100 mg/mL) |
| sodium benzoate | 0.2 g (2.0 mg/mL) | 0.2 g (2.0 mg/mL) |
| xanthan gum | - | 1.0 g (10 mg/mL) |
| sodium chloride | 2.0 g (20 mg/mL) | - |
| sucralose | 0.2 g (2.0 mg/mL) | - |
| stevia | 0.2 g (2.0 mg/mL) | - |
| apple flavor | 0.04 g (0.4 mg/mL) | - |
| erythritol | 30.0 g (300 mg/mL) | - |
| purified water | 100 mL in total | 100 mL in total |

**[Table 42]**

| Formulation amount (Content) | Example 28 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | - | 10.0 g (100 mg/mL) |
| sodium benzoate | 0.2 g (2.0 mg/mL) | 0.2 g (2.0 mg/mL) |
| xanthan gum | - | 1.7 g (17 mg/mL) |
| sodium chloride | 2.0 g (20 mg/mL) | - |
| sucralose | 0.2 g (2.0 mg/mL) | - |
| stevia | 0.2 g (2.0 mg/mL) | - |
| apple flavor | 0.04 g (0.4 mg/mL) | - |
| purified water | 100 mL in total | 100 mL in total |

**[Table 43]**

| Formulation amount (Content) | Example 29 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | - | 10.0 g (100 mg/mL) |
| sodium benzoate | 0.2 g (2.0 mg/mL) | 0.2 g (2.0 mg/mL) |
| xanthan gum | - | 1.8 g (18 mg/mL) |
| sodium chloride | 2.0 g (20 mg/mL) | - |
| sucralose | 0.2 g (2.0 mg/mL) | - |
| stevia | 0.2 g (2.0 mg/mL) | - |
| apple flavor | 0.04 g (0.4 mg/mL) | - |
| purified water | 100 mL in total | 100 mL in total |

The formulation amount and content of each ingredient in the preparations of the above Examples are shown in Table below.

**[Table 44]**

| Formulation amount (Content) | Example 27 | Example 28 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| sodium benzoate | 0.4 g (2.0 mg/mL) | 0.4 g (2.0 mg/mL) |
| xanthan gum | 1.0 g (5 mg/mL) | 1.7 g (8.5 mg/mL) |
| sodium chloride | 2.0 g (10 mg/mL) | 2.0 g (10 mg/mL) |
| sucralose | 0.2 g (1.0 mg/mL) | 0.2 g (1.0 mg/mL) |
| stevia | 0.2 g (1.0 mg/mL) | 0.2 g (1.0 mg/mL) |
| apple flavor | 0.04 g (0.2 mg/mL) | 0.04 g (0.2 mg/mL) |
| erythritol | 30.0 g (150 mg/mL) | - |
| purified water | 200 mL in total | 200 mL in total |
| pH | 4.64 | 4.49 |

**[Table 45]**

| Formulation amount (Content) | Example 29 |
|---|---|
| Compound 1 hydrochloride | 8.0 g (40 mg/mL) |
| hypromellose | 2.0 g (10 mg/mL) |
| propylene glycol | 10.0 g (50 mg/mL) |
| sodium benzoate | 0.4 g (2.0 mg/mL) |
| xanthan gum | 1.8 g (9 mg/mL) |
| sodium chloride | 2.0 g (10 mg/mL) |
| sucralose | 0.2 g (1.0 mg/mL) |
| stevia | 0.2 g (1.0 mg/mL) |
| apple flavor | 0.04 g (0.2 mg/mL) |
| purified water | 200 mL in total |
| pH | 4.46 |

### Comparative Examples 1 and 2

According to the following procedure, the preparations of Comparative Examples 1 and 2 were prepared from Compound 1 hydrochloride, an additive and a solvent in the amounts shown in Table below.

### (1) Preparation of Solution A

Hypromellose, sodium benzoate, and propylene glycol were added to purified water and dissolved. In Comparative Example 1, benzalkonium chloride was further added thereto and dissolved. To each solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

### (2) Preparation of Solution B

Sodium benzoate and propylene glycol were added to purified water and dissolved. In Comparative Example 1, benzalkonium chloride was further added thereto and dissolved. Each solution was then warmed to 70°C, and xanthan gum was added thereto and dissolved. The solution was then cooled to room temperature to prepare Solution B.

### (3) Preparation of Comparative Examples 1 and 2

Solution A and Solution B were mixed in a mass ratio of 1:1 to prepare the preparations of Comparative Examples 1 and 2.

### (4) pH measurement of Comparative Examples 1 and 2

The pH of each preparation of Comparative Examples 1 and 2 was measured according to the method described in the general test method in the Japanese Pharmacopoeia 17th Edition.

**[Table 46]**

| Formulation amount (Content) | Comparative Example 1 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 1.0 g (10 mg/mL) |
| benzalkonium chloride | 1.0 g (10 mg/mL) | 1.0 g (10 mg/mL) |
| purified water | 100 mL in total | 100 mL in total |

**[Table 47]**

| Formulation amount (Content) | Comparative Example 2 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | 5.0 g (50 mg/mL) | 5.0 g (50 mg/mL) |
| sodium benzoate | 0.2 g (2.0 mg/mL) | 0.2 g (2.0 mg/mL) |
| xanthan gum | - | 1.0 g (10 mg/mL) |
| purified water | 100 mL in total | 100 mL in total |

The formulation amount and content of each ingredient in the preparations of the above Comparative Examples are shown in Table below.

**[Table 48]**

| Formulation amount (Content) | Comparative Example 1 | Comparative Example 2 |
|---|---|---|
| Compound 1 hydrochloride | 8.0 (40 mg/mL) g | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| sodium benzoate | 0.4 g (2.0 mg/mL) | 0.4 g (2.0 mg/mL) |
| xanthan gum | 1.0 g (5.0 mg/mL) | 1.0 g (5.0 mg/mL) |
| benzalkonium chloride | 2.0 g (10 mg/mL) | - |
| purified water | 200 mL in total | 200 mL in total |
| pH | 3.65 | 3.53 |

### Comparative Examples 3 and 4

According to the following procedure, the preparations of Comparative Examples 3 and 4 were prepared from Compound 1 hydrochloride, an additive and a solvent in the amounts shown in Table below.

### (1) Preparation of Solution A

Methylparaben was added to purified water at about 70°C and dissolved, and then the solution was cooled to room temperature. Hypromellose, dipotassium phosphate, sucralose, and propylene glycol were then added thereto and dissolved. In Comparative Example 4, sodium chloride was further added thereto and dissolved. To each solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

### (2) Preparation of Solution B

Methylparaben was added to purified water at about 70°C and dissolved, and then propylene glycol and dipotassium phosphate were added thereto and dissolved. In Comparative Example 4, sodium chloride was further added and dissolved. Xanthan gum was then added thereto and dissolved. The solution was then cooled to room temperature to prepare Solution B.

### (3) Preparation of Comparative Examples 3 and 4

Solution A and Solution B were mixed in a mass ratio of 1:1 to prepare the preparations of Comparative Examples 3 and 4.

### (4) pH measurement of Comparative Examples 3 and 4

The pH of each preparation of Comparative Examples 3 and 4 was measured according to the method described in the general test method in the Japanese Pharmacopoeia 17th Edition.

**[Table 49]**

| Formulation amount (Content) | Comparative Example 3 | |
|---|---|---|
| | Solution A | Solution B |
| Compound 1 | 8.0 g (80 mg/mL) | - |
| hydrochloride | | |
| hypromellose | 3.0 g (30 mg/mL) | - |
| propylene glycol | 5.0 g (50 mg/mL) | 5.0 g (50 mg/mL) |
| methylparaben | 0.25 g (2.5 mg/mL) | 0.25 g (2.5 mg/mL) |
| xanthan gum | - | 4.0 g (8.0 mg/mL) |
| sucralose | 0.03 g (0.3 mg/mL) | - |
| dipotassium phosphate | 1.56 g (15.6 mg/mL) | 1.56 g (15.6 mg/mL) |
| purified water | 100 mL in total | 100 mL in total |

**[Table 50]**

| Formulation amount (Content) | Comparative Example 4 | |
|---|---|---|
| | Solution A | Solution B |
| Compound 1 hydrochloride | 8.0 g (80 mg/mL) | - |
| hypromellose | 3.0 g (30 mg/mL) | - |
| propylene glycol | 5.0 g (50 mg/mL) | 5.0 g (50 mg/mL) |
| methylparaben | 0.25 g (2.5 mg/mL) | 0.25 g (2.5 mg/mL) |
| xanthan gum | - | 4.0 g (8.0 mg/mL) |
| sucralose | 0.03 g (0.3 mg/mL) | - |
| dipotassium phosphate | 1.56 g (15.6 mg/mL) | 1.56 g (15.6 mg/mL) |
| sodium chloride | 1.0 g (10 mg/mL) | 1.0 g (10 mg/mL) |
| purified water | 100 mL in total | 100 mL in total |

The formulation amount and content of each ingredient in the preparations of the above Comparative Examples are shown in Table below.

**[Table 51]**

| Formulation amount (Content) | Comparative Example 3 | Comparative Example 4 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| methylparaben | 0.5 g (2.5 mg/mL) | 0.5 g (2.5 mg/mL) |
| xanthan gum | 0.8 g (4.0 mg/mL) | 0.8 g (4.0 mg/mL) |
| sucralose | 0.03 g (0.15 mg/mL) | 0.03 g (0.15 mg/mL) |
| dipotassium phosphate | 3.12 g (15.6 mg/mL) | 3.12 g (15.6 mg/mL) |
| sodium chloride | - | 2.0 g (10 mg/mL) |
| purified water | 200 mL in total | 200 mL in total |
| pH | 6.06 | 6.00 |

### Comparative Example 5

According to the following procedure, the preparation of Comparative Example 5 was prepared from Compound 1 hydrochloride, an additive and a solvent in the amounts shown in Table below.

### (1) Preparation of Solution A

Methylparaben was added to purified water at about 70°C and dissolved, and then the solution was cooled to room temperature. Hypromellose, dipotassium phosphate, monopotassium phosphate, and carmellose sodium were then added thereto and dissolved. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

### (2) Preparation of Solution B

Methylparaben was added to purified water at about 70°C and dissolved, and then dipotassium phosphate, monopotassium phosphate, and xanthan gum were added thereto and dissolved. The solution was then cooled to room temperature to prepare Solution B.

### (3) Preparation of Comparative Example 5

Solution A and Solution B were mixed in a mass ratio of 1:1 to prepare the preparations of Comparative Example 5.

### (4) pH measurement of Comparative Example 5

The pH of the preparation of Comparative Example 5 was measured according to the method described in the general test method in the Japanese Pharmacopoeia 17th Edition.

**[Table 52]**

| Formulation amount (Content) | Comparative Example 5 | |
|---|---|---|
| | Solution A | Solution B |
| Compound 1 hydrochloride | 4.0 g (40 mg/mL) | - |
| hypromellose | 1.0 g (10 mg/mL) | - |
| carmellose sodium | 1.0 g (10 mg/mL) | - |
| methylparaben | 0.2 g (2.0 mg/mL) | 0.2 g (2.0 mg/mL) |
| xanthan gum | - | 0.6 g (6.0 mg/mL) |
| dipotassium phosphate | 1.22 g (12.2 mg/mL) | 1.22 g (12.2 mg/mL) |
| monopotassium phosphate | 0.41 g (4.1 mg/mL) | 0.41 g (4.1 mg/mL) |
| purified water | 100 mL in total | 100 mL in total |

The formulation amount and content of each ingredient in the preparation of the above Comparative Example are shown in Table below.

**[Table 53]**

| Formulation amount (Content) | Comparative Example 5 |
|---|---|
| Compound 1 hydrochloride | 4.0 g (20 mg/mL) |
| hypromellose | 1.0 g (5.0 mg/mL) |
| carmellose sodium | 1.0 g (5.0 mg/mL) |
| methylparaben | 0.4 g (2.0 mg/mL) |
| xanthan gum | 0.6 g (3.0 mg/mL) |
| dipotassium phosphate | 2.44 g (12.2 mg/mL) |
| monopotassium phosphate | 0.82 g (4.1 mg/mL) |
| purified water | 200 mL in total |
| pH | 6.73 |

### Test Example 1 (Measurement of amount of Compound 1 or salt thereof dissolved in 1st fluid for dissolution test)

According to the dissolution test (the paddle method) described in the Japanese Pharmacopoeia 17th Edition, the test was performed under the following [Test Condition]. The test solution was collected by liquid chromatography and the amount of Compound 1 or salt thereof dissolved in the solution was quantified.

### [Test Condition]

Test solution: 1st fluid for dissolution test described in the Japanese Pharmacopoeia 17th Edition
Volume of 1st fluid for dissolution test: 250 mL Temperature of test solution: 36.5 - 37.5°C
Rotation speed: 50 rpm
Test preparation: Preparation comprising 40 mg of "Compound 1 or salt thereof" in terms of Compound 1 hydrochloride Sampling time of test solution: 10 minutes, 20 minutes and 30 minutes from the start of the test

**[Table 54]**

| | Time after the start of test (minute) | | |
|---|---|---|---|
| | Amount of Compound 1 or salt thereof dissolved in 1st fluid for dissolution test (µg/mL) | | |
| | 10 | 20 | 30 |
| Example 1 | 82 | 87 | 86 |
| Example 2 | 83 | 88 | 87 |
| Example 3 | 83 | 88 | 85 |
| Example 4 | 87 | 91 | 89 |
| Example 5 | 82 | 88 | 87 |
| Comparative Example 1 | 90 | 91 | 93 |
| Comparative Example 2 | 87 | 91 | 93 |

**[Table 55]**

| | Time after the start of test (minute) | | |
|---|---|---|---|
| | Amount of Compound 1 or salt thereof dissolved in 1st fluid for dissolution test (µg/mL) | | |
| | 10 | 20 | 30 |
| Example 6 | 60 | 77 | 84 |
| Example 7 | 66 | 82 | 87 |
| Example 8 | 59 | 82 | 87 |
| Example 9 | 59 | 78 | 85 |
| Example 10 | 62 | 82 | 89 |
| Example 11 | 69 | 85 | 89 |
| Example 12 | 69 | 82 | 87 |
| Example 13 | 68 | 83 | 87 |

**[Table 56]**

| | Time after the start of test (minute) | | |
|---|---|---|---|
| | Amount of Compound 1 or salt thereof dissolved in 1st fluid for dissolution test (µg/mL) | | |
| | 10 | 20 | 30 |
| Example 14 | 84 | 89 | 92 |
| Example 15 | 83 | 87 | 90 |
| Comparative Example 3 | 134 | 130 | 128 |
| Comparative Example 4 | 135 | 132 | 131 |

**[Table 57]**

| | Time after the start of test (minute) | | |
|---|---|---|---|
| | Amount of Compound 1 or salt thereof dissolved in 1st fluid for dissolution test (µg/mL) | | |
| | 10 | 20 | 30 |
| Example 16 | 84 | 87 | 88 |
| Example 17 | 84 | 89 | 88 |
| Example 18 | 83 | 88 | 90 |
| Example 19 | 84 | 90 | 91 |

**[Table 58]**

| | Time after the start of test (minute) | | |
|---|---|---|---|
| | Amount of Compound 1 or salt thereof dissolved in 1st fluid for dissolution test (µg/mL) | | |
| | 10 | 20 | 30 |
| Example 20 | 64 | 72 | 75 |
| Example 21 | 34 | 59 | 72 |
| Example 22 | 75 | 80 | 82 |
| Example 23 | 75 | 80 | 81 |
| Comparative Example 5 | 131 | 132 | 132 |

### Test Example 2 (Measurement of concentration of Compound 1 or salt thereof dissolved in preparation (dissolution fractional concentration in preparation))

The suspended particle in preparation was completely precipitated with an ultracentrifuge (150,000 rpm, 10 minutes). When the suspended particle was not precipitated in one operation, the centrifugation was repeated until the suspended particle was completely precipitated. After observing that the particle was precipitated, the dissolution fractional concentration of Compound 1 or salt thereof in the supernatant solution was quantified by liquid chromatography.

**[Table 59]**

| | Dissolution fractional concentration of Compound 1 or salt thereof in preparation (µg/mL) |
|---|---|
| Example 1 | 76 |
| Example 2 | 93 |
| Example 3 | 125 |
| Example 4 | 122 |
| Example 5 | 95 |
| Comparative Example 1 | 438 |
| Comparative Example 2 | 522 |

**[Table 60]**

| | Dissolution fractional concentration of Compound 1 or salt thereof in preparation (µg/mL) |
|---|---|
| Example 6 | 158 |
| Example 7 | 93 |
| Example 8 | 47 |
| Example 9 | 119 |
| Example 1 0 | 117 |
| Example 11 | 82 |
| Example 12 | 101 |
| Example 13 | 79 |

**[Table 61]**

| | Dissolution fractional concentration of Compound 1 or salt thereof dissolved in preparation (µg/mL) |
|---|---|
| Example 14 | 346 |
| Example 15 | 173 |
| Comparative Example 3 | 0 |

**[Table 62]**

| | Dissolution fractional concentration of Compound 1 or salt thereof dissolved in preparation (µg/mL) |
|---|---|
| Example 16 | 79 |
| Example 17 | 76 |
| Example 18 | 93 |
| Example 19 | 91 |

**[Table 63]**

| | Dissolution fractional concentration of Compound 1 or salt thereof dissolved in preparation (µg/mL) |
|---|---|
| Example 20 | 90 |
| Example 21 | 105 |
| Example 22 | 81 |
| Example 23 | 79 |
| Comparative Example 5 | 0 |

### Test Example 3 (Measurement of particle size distribution of Compound 1 or salt thereof)

40 mL of a solvent in which Compound 1 or salt thereof cannot be dissolved (purified water or neutral buffer solution) was added to about 0.01 mL of an aqueous suspension preparation to prepare a measurement solution. The particle size distribution in the measurement solution was measured with laser diffraction particle size analyzer (HELOS/BR-MULTI) to calculate the D50 and D90 values.

**[Table 64]**

| | D50 (µm) | D90 (µm) |
|---|---|---|
| Example 1 | 2.70 | 6.59 |
| Example 2 | 2.50 | 5.54 |
| Example 3 | 2.49 | 5.59 |
| Example 4 | 2.40 | 6.88 |
| Example 5 | 2.39 | 5.64 |
| Comparative Example 1 | 102.6 | 163.4 |
| Comparative Example 2 | 14.38 | 35.07 |

**[Table 65]**

| | D50 (µm) | D90 (µm) |
|---|---|---|
| Example 6 | 2.71 | 5.98 |
| Example 7 | 2.72 | 6.01 |
| Example 8 | 2.64 | 5.51 |
| Example 9 | 2.76 | 5.86 |
| Example 10 | 2.48 | 5.36 |
| Example 11 | 2.47 | 5.22 |
| Example 12 | 2.71 | 5.72 |
| Example 13 | 2.50 | 5.21 |

**[Table 66]**

| | D50 (µm) | D90 (µm) |
|---|---|---|
| Example 14 | 2.89 | 6.37 |
| Example 15 | 2.53 | 5.53 |
| Comparative Example 3 | 3.74 | 6.54 |

**[Table 67]**

| | D50 (µm) | D90 (µm) |
|---|---|---|
| Example 20 | 2.83 | 6.61 |
| Example 21 | 2.70 | 6.08 |
| Example 22 | 2.69 | 6.00 |
| Example 23 | 2.69 | 5.66 |
| Comparative Example 5 | 5.05 | 8.56 |

### Test Example 4 (Measurement of particle size distribution of Compound 1 or salt thereof)

40 mL of a solvent in which Compound 1 or salt thereof cannot be dissolved (purified water or neutral buffer solution) was added to each aqueous suspension preparation of Examples 24-29 (about 0.01 mL) to prepare measurement solutions. The particle size distribution in each measurement solution was measured with laser diffraction particle size analyzer (HELOS/BR-MULTI) to calculate the D50 and D90 values.

**[Table 68]**

| | D50 (µm) | D90 (µm) |
|---|---|---|
| Example 24 | 2.61 | 5.63 |
| Example 25 | 2.71 | 5.84 |
| Example 26 | 2.78 | 6.27 |
| Example 27 | 2.80 | 6.44 |
| Example 28 | 2.63 | 5.74 |
| Example 29 | 2.70 | 5.65 |

### Test Example 5 (Measurement of amount of Compound 1 or salt thereof dissolved in 1st fluid for dissolution test)

According to the dissolution test (the paddle method) described in the Japanese Pharmacopoeia 17th Edition, the aqueous suspension preparations of Examples 24-29 were tested under the following [Test Condition]. The test solutions were collected by liquid chromatography and the amount of Compound 1 or salt thereof dissolved in each solution was quantified.

### [Test Condition]

Test solution: 1st fluid for dissolution test described in the Japanese Pharmacopoeia 17th Edition
Volume of 1st fluid for dissolution test: 250 mL Temperature of test solution: 36.5 - 37.5°C
Rotation speed: 50 rpm
Test preparation: Preparation comprising 40 mg of "Compound 1 or salt thereof" in terms of Compound 1 hydrochloride Sampling time of test solution: 10 minutes, 20 minutes and 30 minutes after the start of test

**[Table 69]**

| | Time after the start of test (minute) | | |
|---|---|---|---|
| | Amount of Compound 1 or salt thereof dissolved in 1st fluid for dissolution test (µg/mL) | | |
| | 10 | 20 | 30 |
| Example 24 | 64.7 | 80.3 | 86.5 |
| Example 25 | 79.0 | 85.5 | 86.8 |
| Example 26 | 69.3 | 81.3 | 86.3 |
| Example 27 | 94.6 | 94.4 | 94.9 |
| Example 28 | 74.4 | 83.2 | 89.4 |
| Example 29 | 64.9 | 78.4 | 86.3 |

### INDUSTRIAL APPLICABILITY

The present invention can provide a high quality aqueous suspension preparation which exhibits the solubility in the stomach depending on the property of a drug without producing rapid dissolution and absorption even when the aqueous suspension pharmaceutical preparation comprising Compound 1 or salt thereof as the active ingredient is administered orally and can inhibit the particle growth and the aggregation of particles.

## Claims

1. An aqueous suspension pharmaceutical preparation with a pH of 2.5 - 5.5, comprising the following (1) to (4):
(1) (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1), a pharmaceutically acceptable acid addition salt thereof, or a mixture thereof,
(2) one or more chlorides selected from an inorganic chloride or a quaternary ammonium chloride having 4 to 12 carbon atoms,
(3) a dispersant, and
(4) water.

2. The pharmaceutical preparation according to claim 1, wherein the pH is 3.0 - 5.5.

3. The pharmaceutical preparation according to claim 1, wherein the pH is 3.0 - 5.0.

4. The pharmaceutical preparation according to any one of claims 1 to 3, which comprises at least one ingredient selected from the group consisting of a cellulose derivative, a sucrose fatty acid ester, and polyvinyl alcohol as the dispersant.

5. The pharmaceutical preparation according to claim 4, which comprises a cellulose derivative as the dispersant.

6. The pharmaceutical preparation according to any one of claims 1 to 3, which comprises at least one ingredient selected from the group consisting of hypromellose, hydroxypropylcellulose, and hydroxyethyl cellulose as the dispersant.

7. The pharmaceutical preparation according to claim 6, which comprises hypromellose as the dispersant.

8. The pharmaceutical preparation according to any one of claims 1 to 7, which comprises at least one ingredient selected from the group consisting of sodium chloride, potassium chloride, iron (II) chloride, calcium chloride, magnesium chloride, and choline chloride as the chloride.

9. The pharmaceutical preparation according to claim 8, which comprises at least one ingredient selected from the group consisting of sodium chloride, potassium chloride, and choline chloride as the chloride.

10. The pharmaceutical preparation according to claim 8, which comprises at least one ingredient selected from the group consisting of sodium chloride and choline chloride as the chloride.

11. The pharmaceutical preparation according to claim 8, which comprises sodium chloride as the chloride.

12. The pharmaceutical preparation according to any one of claims 1 to 11, wherein the concentration of chloride ion is about 0.015 - about 1 mol/L, provided that the chloride ion does not comprise the chloride ion derived from a salt of Compound 1.

13. The pharmaceutical preparation according to claim 12, wherein the concentration of chloride ion is about 0.05 - about 1 mol/L.

14. The pharmaceutical preparation according to claim 12, wherein the concentration of the chloride ion is about 0.10 - about 1 mol/L.

15. The pharmaceutical preparation according to any one of claims 1 to 14, wherein the content of Compound 1, a pharmaceutically acceptable acid addition salt thereof, or a mixture thereof is about 5 - about 120 mg/mL in terms of Compound 1 hydrochloride.

16. The pharmaceutical preparation according to any one of claims 1 to 14, wherein the content of Compound 1, a pharmaceutically acceptable acid addition salt thereof, or a mixture thereof is about 10 - about 100 mg/mL in terms of Compound 1 hydrochloride.

17. The pharmaceutical preparation according to any one of claims 1 to 14, wherein the content of Compound 1, a pharmaceutically acceptable acid addition salt thereof, or a mixture thereof is about 20 - about 80 mg/mL in terms of Compound 1 hydrochloride.

18. The pharmaceutical preparation according to any one of claims 1 to 17, wherein Compound 1, a pharmaceutically acceptable acid addition salt thereof, or a mixture thereof is Compound 1 hydrochloride.

19. The pharmaceutical preparation according to any one of claims 1 to 18 for oral administration.

20. The pharmaceutical preparation according to claims 1 to 19, wherein the maximum dissolution amount of Compound 1, a pharmaceutically acceptable acid addition salt thereof, or
a mixture thereof at 0 - 30 minutes after the start of the test performed under the following test condition according to the dissolution test (the paddle method) described in the Japanese Pharmacopoeia 17th Edition is about 120 µg/mL or less in terms of Compound 1 hydrochloride.
[Test Condition]
Test solution: 1st fluid for dissolution test described in the Japanese Pharmacopoeia 17th Edition
Volume of 1st fluid for dissolution test: 250 mL Temperature of test solution: 36.5 - 37.5°C Rotation speed: 50 rpm
Test preparation: Preparation comprising 40 mg of Compound 1, a pharmaceutically acceptable acid addition salt thereof, or a mixture thereof in terms of Compound 1 hydrochloride Sampling time of test solution: 10 minutes, 20 minutes and 30 minutes after the start of test

21. The pharmaceutical preparation according to claim 20, wherein the maximum dissolution amount of Compound 1, a pharmaceutically acceptable acid addition salt thereof, or a mixture thereof at 0 - 30 minutes after the start of the test is about 110 µg/mL or less in terms of Compound 1 hydrochloride.

22. The pharmaceutical preparation according to claim 20, wherein the maximum dissolution amount of Compound 1, a pharmaceutically acceptable acid addition salt thereof, or a mixture thereof at 0 - 30 minutes after the start of the test is about 100 µg/mL or less in terms of Compound 1 hydrochloride.

23. The pharmaceutical preparation according to any one of claims 1 to 22, wherein the D90 of the suspended particle is about 1 to about 30 µm.

24. The pharmaceutical preparation according to any one of claims 1 to 22, wherein the D90 of the suspended particle is about 1 to about 20 µm.

25. The pharmaceutical preparation according to any one of claims 1 to 22, wherein the D90 of the suspended particle is about 1 to about 15 µm.

26. The pharmaceutical preparation according to any one of claims 1 to 22, wherein the D50 of the suspended particle is about 0.1 to about 13 µm.

27. The pharmaceutical preparation according to any one of claims 1 to 22, wherein the D50 of the suspended particle is about 0.1 to about 10 µm.

28. The pharmaceutical preparation according to any one of claims 1 to 22, wherein the D50 of the suspended particle is about 0.1 to about 7 µm.

29. The pharmaceutical preparation according to any one of claims 1 to 28, wherein the concentration of Compound 1, a pharmaceutically acceptable acid addition salt thereof, or a mixture thereof dissolved in the preparation (the dissolution fractional concentration in the preparation) is about 360 µg/mL or less in terms of Compound 1 hydrochloride.

30. The pharmaceutical preparation according to any one of claims 1 to 28, wherein the concentration of Compound 1, a pharmaceutically acceptable acid addition salt thereof, or a mixture thereof dissolved in the preparation (the dissolution fractional concentration in the preparation) is about 250 µg/mL or less in terms of Compound 1 hydrochloride.

31. The pharmaceutical preparation according to any one of claims 1 to 28, wherein the concentration of Compound 1, a pharmaceutically acceptable acid addition salt thereof, or a mixture thereof dissolved in the preparation (the dissolution fractional concentration in the preparation) is about 200 µg/mL or less in terms of Compound 1 hydrochloride.

32. The pharmaceutical preparation according to any one of claims 1 to 28, wherein the concentration of Compound 1, a pharmaceutically acceptable acid addition salt thereof, or a mixture thereof dissolved in the preparation (the dissolution fractional concentration in the preparation) is about 175 µg/mL or less in terms of Compound 1 hydrochloride.

33. A medicament for treatment and/or prophylaxis of a psychiatric disease, which comprises the pharmaceutical preparation according to any one of claims 1 to 32.

34. The medicament according to claim 33, wherein the psychiatric disease is schizophrenia, bipolar disorder, senile dementia, or depression.

35. A method of treating and/or preventing a psychiatric disease, which comprises administering the pharmaceutical preparation according to any one of claims 1 to 32 to a patient in need thereof.

36. The method according to claim 35, wherein the psychiatric disease is schizophrenia, bipolar disorder, senile dementia, or depression.

37. Use of the pharmaceutical preparation according to any one of claims 1 to 32 in the manufacture of a medicament for treatment and/or prophylaxis of a psychiatric disease.

38. The use according to claim 37, wherein the psychiatric disease is schizophrenia, bipolar disorder, senile dementia, or depression.

39. The pharmaceutical preparation according to any one of claims 1 to 32 for use in the treatment and/or prophylaxis of a psychiatric disease.

40. The pharmaceutical preparation according to claim 39, wherein the psychiatric disease is schizophrenia, bipolar disorder, senile dementia, or depression.
